(19) Europäisches Patentamt
European Patent Office
Office européen des brevets

(11) Veröffentlichungsnummer: **0 402 485 A1**

(12) # EUROPÄISCHE PATENTANMELDUNG veröffentlicht nach Art. 158 Abs. 3 EPÜ

(21) Anmeldenummer: **90901095.1**

(22) Anmeldetag: **22.12.89**

(86) Internationale Anmeldenummer:
**PCT/SU89/00331**

(87) Internationale Veröffentlichungsnummer:
**WO 90/07488 (12.07.90 90/16)**

(51) Int. Cl.5: **C07C 205/12, C07C 201/08, C07C 201/06, C07C 201/14, C07B 39/00, C07B 43/02**

(30) Priorität: **30.12.88 SU 4622749**

(43) Veröffentlichungstag der Anmeldung:
**19.12.90 Patentblatt 90/51**

(84) Benannte Vertragsstaaten:
**CH DE GB IT LI**

(71) Anmelder: **ANDRIEVSKY, Alexandr Mikhailovich**
**ul. Sivashskaya, 4-3-100**
**Moscow, 113149(SU)**

Anmelder: **GORELIK, Mikhail Viktorovich**
**ul. Pervomaiskaya 46-60 Moskovskaya obl.**
**Dolgoprudny, 141700(SU)**

Anmelder: **AVIDON, Sergei Viktorovich**
**ul. Godovikova, 1-2-72**
**Moscow, 129085(SU)**

Anmelder: **GORDIEVSKAYA, Evgenia Vasilievna**
**ul. Presnensky Val, 42-7**
**Moscow, 123557(SU)**

Anmelder: **ALTMAN, Elena Shulimovna**
**Petrovsko-Razumovsky proezd, 7-14**
**Moscow 125083(SU)**

Anmelder: **VOROZHTSOV, Georgy Nikolaevich**
**ul. Sadovaya-Spasskaya, 21-208**
**Moscow, 107078(SU)**

Anmelder: **DJUMAEV, Kirill Mikhailovich**
**ul. B. Filevskaya, 55-2-27**
**Moscow, 121433(SU)**

(72) Erfinder: **ANDRIEVSKY, Alexandr Mikhailovich**
**ul. Sivashskaya, 4-3-100**
**Moscow, 113149(SU)**
Erfinder: **GORELIK, Mikhail Viktorovich**
**ul. Pervomaiskaya 46-60 Moskovskaya obl.**
**Dolgoprudny, 141700(SU)**
Erfinder: **AVIDON, Sergei Viktorovich**
**ul. Godovikova, 1-2-72**
**Moscow, 129085(SU)**
Erfinder: **GORDIEVSKAYA, Evgenia Vasilievna**
**ul. Presnensky Val, 42-7**
**Moscow, 123557(SU)**
Erfinder: **ALTMAN, Elena Shulimovna**
**Petrovsko-Razumovsky proezd, 7-14**
**Moscow 125083(SU)**
Erfinder: **VOROZHTSOV, Georgy Nikolaevich**
**ul. Sadovaya-Spasskaya, 21-208**
**Moscow, 107078(SU)**
Erfinder: **DJUMAEV, Kirill Mikhailovich**
**ul. B. Filevskaya, 55-2-27**
**Moscow, 121433(SU)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **VERFAHREN ZUR HERSTELLUNG VON 2-BROM-4,6-DINITROCHLORBENZOL.**

EP 0 402 485 A1

Xerox Copy Centre

(57) Die Herstellung von 2-Brom-4,6-dinitrochlorbenzol wird aus Chlorbenzol, das vorher mit einem Gemisch aus Schwefel- und Salpetersäure behandelt wird, oder aus individuellen o-, p-Nitrochlorbenzolen, oder aus einem eutektischen Gemisch von o-, p-, m-Nitrochlorbenzolen vorgenommen, indem man beliebige der genannten Stoffe bei einer Temperatur von 20 bis 120 °C mit Brom oder mit Alkalimetallbromid, mit Salpetersäure oder mit Alkalimetallnitrat und mit Schwefelsäure oder mit Oleum bei folgenden Molverhältnissen der Reagenzien behandelt: Ausgangsstoff: Brom oder Alkalimetallbromid: Salpetersäure oder Alkalimetallnitrat: Schwefelsäure oder Oleum gleich 1,0:0,5-1,5 oder 1,0-3,0:2,0-4,0 oder 2,5-5,0:6,0-70,0.

EPAA-39731.9

# VERFAHREN ZUR HERSTELLUNG VON 2-BROM-4,6-DINITROCHLORBENZOL

## Gebiet der Technik

Die angemeldete Erfindung bezieht sich auf organische Synthese und insbesondere betrifft sie ein Verfahren zur Herstellung von 2-Brom-4,6-dinitrochlorbenzol.

## Zugrundeliegender Stand der Technik

Gegenwärtig sind zwei Hauptmethoden bekannt, die die Herstellung von 2-Brom-4,6-dinitrochlorbenzol folgender Formel (I):

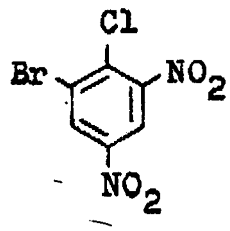

in einer ausreichend reinen Form gewährleisten, das heisst isomerfrei.

So wird in DE, C3, 2657234 ein Verfahren beschrieben, das aus der Durchführung der Sandmeyer-Reaktion unter Verwendung von 2,4-dinitro-6-bromanilin beruht. In Übereinstimmung mit diesem Verfahren nimmt man zuerst die Umsetzung des Anilins mit einem Halogenagens vor, indem Halogenanilin anfällt, das im weiteren der Umsetzung mit Alkoholen oder mit nitrosierenden Agenzien in Gegenwart von Wasser und Säure ausgesetzt wird.

Dabei fallen als Nebenprodukte Phenolester der entsprechenden Alkohole mit einer grösseren oder kleineren Mengen an Harzen an.

Die Ausbeute und der Reinheitsgrad der anfallenden Produkte sind unzufriedenstellend, insbesondere bei der grosstechnischen Produktion.

In DE, B2, 2001570 ist ein Verfahren zur Herstellung von 2-Brom-4,6-dinitrochlorbenzol beschrieben, das auf einer Austauschreaktion des 2-4-Dinitro-6-beomphenols mit einem Halogenagens. Thionilchlorid im Dimethylformamid, beruht. Das genannte Verfahren hat keine grosstechnische Anwendung im Zusammenhang damit gefunden, weil das Reaktionsgemisch nach der Beendigung der Austauschreaktion ein teerartiges Harz darstellt, aus dem das Endprodukt, wenn auch isomerfrei, aber wesentlich verunreinigt, mit grossen

Schwierigkeiten ausgeschieden werden kann.

Ein vollkommeneres Verfahren, beruhend auf der Methode der Halogenierung von Phenolen, stellt ein Verfahren dar, das in DD, A5, 204246 beschrieben wird, gemässe dem man das Endprodukt durch die Behandlung des Dinitrodihalogenphenols mit Chlormethylendimethyliminotrichlorphosphat erhält, das durch Umsetzung von I Mol Phosphor (V) oxidchlorids mit I Mol Dimethylformamid gewonnen wird. Die Ausbeute an Endprodukt beträgt etwa 70%.

Auf Grund dieses Verfahrens wurde es im Vergleich zu den anderen Verfahren möglich, die Menge von Abfällen zur Hälfte zu reduzieren.

Für alle genannten Verfahren sind jedoch charakteristisch:

- hohe materielle Aufwendungen infolge der Notwendigkeit, Schutzmassnahmen gegen die schädliche Einwirkung der verwendbaren Reagenzien auf die Umwelt vornehmen zu müssen;

- die Notwendigkeit bei der Verwendung von inerten Lösungsmitteln die Zersetzung des Überschusses an einem Halogenagens mit Wasser und im weiteren die Abscheidung der organischen Phase von der wässerigen Phase oder die gemeinsame Destillation mit Dampf vornehmen zu müssen.

Die Verwendung des Überschusses an saueren Halogenverbindungen unter Verzicht auf die Nutzung von Lösungsmitteln verlangt auch die Regeneration des ungenutzten Teils derselben oder die Zersetzung dieses Überschusses mit Wasser. In diesem Fall entsteht eine wesentliche Menge von nichtverwendbaren korrosionsaktiven und die Umwelt verschmutzenden Produkten.

Offenbarung der Erfindung

Der vorliegenden Erfindung liegt die Aufgabe zugrunde, durch die gleichzeitige Dinitrierung und Bromierung von Chlorbenzolderivaten ein Verfahren zur Herstellung von 2-Brom-4,6-dinitrochlorbenzol zu entwickeln, welches die Verwendung hochtoxischer Rohstoffe und die Entstehung von Korrosionsabfällen bei einer hohen Ausbeute an Endprodukt ausschliesst.

Die genannte Aufgabe wird dadurch gelöst, dass man im Verfahren zur Herstellung von 2-Brom-4,6-dinitrochlorbenzol aus einer aromatischen Halogenderivat-Verbindung erfindungsgemäss als aromatische Halogenderivat-Verbindung das Chlorbenzol, das vorher mit einem Gemisch der Schwefel- und Salpetersäure behandelt wird, oder individuelle o-, p-, m-Nitrochlorbenzole verwendet, die man der Behandlung mit Brom oder mit Alkalimetallbromid, mit Salpetersäure oder mit Alkalimetallnitrat und mit Schwefelsäure oder mit Oleum bei folgenden Molverhältnissen der Reagenzien aussetzt: die genannte aromatische Halogenderivat-Verbindung: Brom oder Alkalimetallbromid: Salpetersäure oder Alkalimetallnitrat: Schwefelsäure oder Oleum gleich I,0:0,5-I,5 oder I,0:3,0:2,0-4,0 oder 2,5-5,0:6,0-70,0 bei einer Temperatur von 20 bis 80°C.

Das erfindungsgemässe Verfahren schliesst die Verwendung hochtoxischer Rohstoffe und die Entstehung von Korrosionsabfällen aus, die Ausbeute an Endprodukt betragt 99,8%. Dabei ermöglicht es das erfindungsgemässe Verfahren, das als Abfall der Produktion individueller Nitrochlorbenzole auftretende eutektische Gemisch von Isomernitrochlorbenzolen zu verwendet.

Für die Erreichung einer maximal für das erfindungsgemässe Verfahren möglichen Ausbeute an Endprodukt ist es bei der Verwendung als Ausgangsverbindung von Chlorbenzol zweckmässig, das letztere der Behandlung mit einem Gemisch aus Salpeter und Schwefelsäure bei einem Molverhaltnis I,0:I,I:0,9 mit anschliessender Behandlung des anfallenden Reaktionsgemisches mit einem Gemisch des Oleums, Broms und der Salpetersäure bei ihrem Molverhältnis 1,0:6,9:0,5:2,0 auszusetzen.

Für die Erreichung einer maximal für das erfindungsgemässe Verfahren möglichen Ausbeute an Endprodukt bei der Verwendung als Ausgangsverbindung des p-Nitrochlorbenzols ist es zweckmässig, das letztere mit einem Gemisch aus Schwefelsaure, Brom und Salpetersäure bei ihrem Molverhältnis I,0:70,0:I,5:2,5 zu behandeln.

Für die Erreichung einer maximal für das erfindungsge-

mässe Verfahren mögliche Ausbeute an Endprodukt bei der Verwendung als Ausgangsverbindung des o-Nitrochlorbenzols ist es zweckmässig, das letztere mit einem Gemisch aus Schwefelsäure, Brom und Salpetersäure bei ihrem Molverhältnis 1,0:58,0:0,5:2,0 zu behandeln.

Weitere Ziele und Vorteile der beanspruchten Erfindung werden aus der nachstehenden ausführlichen Beschreibung des Verfahrens zur Herstellung von 2-Brom-4,6-dinitrochlorbenzol und aus den konkreten Beispielen der Realisierung dieses Verfahrens ersichtlich.

Das erfindungsgemässe Verfahren zur Herstellung von 2-Brom-4,6-dinitrochlorbenzol beruht auf einer grundsätzlich neuen Methode, auf einer erstmalig entdeckten Reaktion der gleichzeitigen Durchführung der Dinitrierung und Bromierung.

Beste Ausführungsvariante der Erfindung

In Übereinstimmung mit dem erfindungsgemässen Verfahren wird der gleichzeitigen Dinitrierung und Bromierung entweder das Chlorbenzol, das vorher mit einem Gemisch der Salpetersäure und der Schwefelsäure behandelt wird, oder individuelle o-, p-Chlorbenzole, oder das eutektisches Gemisch der p-, o-, m-Nitrochlorbenzole ausgesetzt. Für die Durchführung der genannten Dinitrierung und Bromierung werden die genannten Ausgangsverbindungen mit Brom mit Alkalimetallbromid, mit Salpetersäure oder mit Alkalimetallnitrat und mit Schwefelsäure oder mit Oleum behandelt. Dabei beträgt das Molverhältnis der genannten Reagenzien entsprechend: nächstgenannte aromatische Halogenderivat-Verbindung: Brom oder Alkalimetallbromid: Schwefelsäure oder Oleum gleich 1,0:0,5-1,5 oder 1,0-3,0:2,0-4,0 oder 2,5-5,0:6,0-70,0.

In Übereinstimmung mit der beanspruchten Erfindung wird die gleichzeitige Dinitrierung und Bromierung bei einer Temperatur von 20 bis 80°C vorgenommen.

Bei Verhältnissen unter den genannten Werten kommt der Prozess entweder nicht zustande oder verläuft unzufriedenstellend, die Verhältnisse oberhalb der genannten Werte ermöglichen es nicht, die Erhöhung des Geschwindigkeitseffektes der Prozessführung zu erreichen, und sie werden ökonomisch nicht zweckmässig.

Die Durchführung des erfindungsgemässen Verfahrens bei einer Temperatur oberhalb von 80°C erscheint wirtschaftlich nicht zweckmässig; bei einer Temperatur unter 20°C verläuft die Prozessführung langsam und mit einer niederigen Ausbeute an Endprodukt.

Hiermit wurde es infolge der Realisierung des erfindungsgemässen Verfahrens möglich, das 2-Brom-4,6-dinitrochlorbenzol in einer Ausbeute zu erhalten, die bis 99,8% beträgt; das herzustellende Produkt zeichnet sich durch einen hohen Reinheitsgrad und ist für präparative Zwecke verwendbar. Dabei ist die Verwendung von hochtoxischen Rohstoffen und die Entstehung von Korrosionsabfällen vollständig ausgeschlossen. Das erfindungsgemässe Verfahren ermöglicht es ausserdem das als Abfall der Produktion von individuellen Nitrochlorbenzolen anfallende eutektische Gemisch von Isomernitrochlorbenzolen zu verwerten.

Zur besseren Erläuterung der vorliegenden Erfindung werden nachstehende Beispiele für ihre konkrete Ausführung angeführt.

Beispiel I

3,8 g (0,025 Mol) p-Nitrochlorbenzol löst man in 62 ml (I,I3 Mol) Schwefelsäure (d = I,84) auf, man setzt 2,0 g (0,0I25 Mol) Brom, 2,2 ml (0,05 Mol) Salpetersäure (d=I,505) zu und erwärmt man auf 70°C. Bei dieser Temperatur erfolgt das Vermischen innerhalb von 4 Stunden. Die Reaktionslösung wird abgekühlt und auf I50 g Eis ausgegossen, der Niederschlag wird abgefiltert, mit Wasser gewaschen und getrocknet. Man erhält 7,00 g (99,5%) 2-Brom-4,6-nitrochlorbenzol, hellgelbe Kristalle aus 70%iger wässeriger Essigsäure, Schmelzpunkt beträgt von 6I bis 62°C ($R_f$ 0,43) (Benzol:Hexan = I:I).

Beispiel 2

I,4 g (0,0I Mol) p-Nitrochlorbenzol löst man in 40 ml (0,7 Mol) Schwefelsäure (d = I,83) auf, man setzt 2,4 g (0,0I5 Mol) Brom, I,08 ml (0,025 Mol) Salpetersäure (d= =I,505) zu und vermischt man bei Raumtemperatur innerhalb von 63 Stunden. Die Reaktionsmasse wird auf Eis ausgegossen, der Rückstand wird abgefiltert, mit Wasser gewaschen

und getrocknet. Man erhält 2,67 g (94,8%) 2-Brom-4,6-dinitrochlorbenzol.

Beispiel 3

0,7 g (0,005 Mol) p-Nitrochlorbenzol löst man in 40 ml (0,7 Mol) Schwefelsäure (d = I,83) auf, man setzt 0,8 g (0,005 Mol) Brom, 0,43 ml (0,0I Mol) Salpetersäure (d = = I,505) zu und erwärmt man auf eine Temperatur von I20°C. Bei dieser Temperatur wird die Reaktionslösung innerhalb von 3 Stunden vermischt, dann auf Raumtemperatur abgekühlt und auf Eis ausgegossen. Der Niederschlag wird abgefiltert, mit Wasser gewaschen und getrocknet. Man erhält I,40 g (99,4%) 2-Brom-4,6-dinitrochlorbenzol.

Beispiel 4

47,25 g (0,3 Mol) p-Nitrochlorbenzol löst man in I00 ml (I,8 Mol) Schwefelsäure (d = I,83) auf, man setzt 26,4 g (0,I65 Mol) Brom und tropfenweise 26,0 ml (0,6 Mol) Salpetersäure (d = I,505) zu, die Temperatur unterhält man dabei in einem Bereich von 65 bis 70°C. Dann wird die Reaktionslösung innerhalb von 25 Stunden bei einer Temperatur von 65°C vermischt, abgekühlt und auf Eis ausgegossen, der Niederschlag wird abgefiltert, mit Wasser gewaschen und getrocknet. Man erhält 83,03 g (99,7%) 2-Brom-4,6-dinitrochlorbenzol.

Beispiel 5

I,0 g (0,006 Mol) p-Nitrochlorbenzol löst man in 10 ml (0,7 Mol) Schwefelsäure (d = I,83) auf, man setzt 0,5 g (0,003 Mol) Brom und tropfenweise 0,5 ml (0,0I2 Mol) Salpetersäure (d = I,505) zu, indem man die Temperatur in einem Bereich von 65 bis 70°C unterhält. Dann wird das Vermischen innerhalb von I0 Stunden bei einer Temperatur von 65°C vorgenommen. Die Reaktionsmasse wird auf Eis ausgegossen, der Niederschlag abgefiltert, mit Wasser gewaschen und getrocknet. Man erhält I,68 g (99,4%) 2-Brom-4,6-dinitrochlorbenzol.

Beispiel 6

In 42 ml (0,75 Mol) Schwefelsäure (d = I,839) löst man 2,0 g (0,0I3 Mol) p-Nitrochlorbenzol auf, man setzt I,I2 g (0,007 Mol) Brom und tropfenweise 2,27 ml (0,052 Mol)

Salpetersäure (d = I,505) zu, indem man die Temperatur in einem Bereich von 45 bis 50°C unterhält. Dann wird das Vermischen innerhalb von 6 Stunden bei einer Temperatur von 65 bis 70°C vorgenommen. Nach der Isolierung des Produktes erhält man 3,64 g (99,5%) 2-Brom-4,6-dinitrochlorbenzol.

Beispiel 7

In 42 ml (0,75 Mol) Schwefelsäure (d = I,839) löst man 2,0 g (0,013 Mol) p-Nitrochlorbenzol auf, man setzt 3,I2 g (0,02 Mol) Brom und tropfenweise I,2 ml (0,026 Mol) Salpetersäure zu, indem man die Temperatur in einem Bereich von 45 bis 50°C unterhält. Dann wird die Reaktionslösung innerhalb von 3 Stunden bei einer Temperatur von 65 bis 70°C vermischt. Nach der Isolierung des Produktes erhält man 3,65 g (99,8%) 2-Brom-4,6-dinitrochlorbenzol.

Beispiel 8

I,0 g (0,006 Mol) o-Nitrochlorbenzol löst man in 20ml (0,35 Mol) Schwefelsäure (d = I,83) auf, dann wird die Synthese unter den in Beispiel 5 genannten analogen Bedingungen geführt. Nach der Isolierung des Niederschlags erhält man I,69 g eines Reaktionsproduktes, es wird aus 70%iger wässeriger Essigsäure umkristallisiert und man erhält I,44 g (85,3%) 2-Brom-4,6-dinitrochlorbenzol.

Beispiel 9

In 20 ml (0,35 Mol) Schwefelsäure (d = I,83) löst man I,0 g (0,006 Mol) eines Gemisches der o- und p-Nitrochlorbenzole (I:20,7) auf, das I,0% m-Nitrochlorbenzol aufweist.

Im weiteren wird die Synthese unter den in Beispiel 5 genannten analogen Bedingungen geführt. Nach der Ausscheidung des Niederschlags erhält man I,69 g eines Reaktionsproduktes, es wird aus 70%iger Essigsäure umkristallisiert und man erhält I,46 g (86,4%) 2-Brom-4,6-dinitrochlorbenzol.

Beispiel I0

In 20 ml (0,35 Mol) Schwefelsäure (d = I,83) löst man I,0 g (0,006 Mol) Gemisch der o- und p-Nitrochlorbenzole (I,66:I) auf, das I,0% m-Isomeres aufweist. Im weiteren wird die Synthese unter den in Beispiel 5 genannten analogen Bedingungen durchgeführt. Nach der Isolierung erhält man I,68 g Reaktionsprodukt, es wird aus 70%iger Essig-

säure umkristallisiert; man erhält I,46 g (86,4%) 2-Brom-4,6-dinitrochlorbenzol.

Beispiel II

In 20 ml Schwefelsäure löst man I,0 g (0,006 Mol) eutektisches Gemisch der o-, p-, m-Nitrochlorbenzole mit einem Gehalt an Isomeren: o- 47%, p - 39%, m - I4% auf. Im weiteren wird die Synthese unter den in Beispiel 5 genannten analogen Bedingungen durchgeführt. Nach der Isolierung erhält man I,69 g eines Reaktionsproduktes, es wird dreimal aus 70%iger Essigsäure umkristallisiert; man erhält I,I6 g (68,9%) 2-Brom-4,6-dinitrochlorbenzol.

Beispiel I2

Den I0,I8 ml (0,I Mol) Chlorbenzol setzt man unter Abkühlung I7,08 g nitrierendes Gemisch zu, das 6,49 g (0,II Mol) Salpetersäure (d = I,505), 9,48 g (0,09 Mol) Schwefelsäure d = I,83) und 0,34 g Wasser enthält. Die Temperatur wird in einem Bereich von 60 bis 65°C unterhalten. Dann wird das Reaktionsgemisch auf 75 bis 80°C erwärmt und innerhalb von 0,5 Stunden vermischt.

Danach wird das Reaktionsgemisch auf 60°C abgekühlt, man setzt 66,6 g Oleum (7,6%), 8,0 g (0,05 Mol) Brom und tropfenweise 8,8 ml (0,2 Mol) Salpetersäure (d = I,505) zu. Es wird auf 70°C erwärmt und innerhalb von I8 Stunden vermischt. Danach wird die Reaktionsmasse analog dem Beispiel 2 ausgeschieden. Man erhalt 27,47 g eines Reaktionsproduktes, es wird aus 70%iger Essigsäure umkristallisiert; man erhält 23,38 g (83,06%) 2-Brom-4,6-dinitrochlorbenzol.

Beispiel I3

In 20 ml (0,35 Mol) Schwefelsäure (d = I,83) löst man I,0 g (0,006 Mol) p-Nitrochlorbenzol auf. Im weiteren wird die Reaktion unter den in Beispiel 5 genannten analogen Bedingungen durchgeführt, mit Ausnahme dessen, dass man 0,37 ml (0,009 Mol) Salpetersäure hinzusetzt und das Reaktionsgemisch innerhalb von 20 Stunden stehenlasst. Man erhält I,37 g Gemisch aus 2,4-Dinitrochlorbenzol und 2-Brom-4,6-dinitrochlorbenzol.

Beispiel I4

Einer Lösung aus I,56 g (0,0I Mol) p-Nitrochlorbenzol

in 40 ml Schwefelsäure (d = I,83) setzt man bei einer Temperatur von 40 bis 50°C 2,4 g (0,0I5 Mol) Brom und 3,24 g (0,033 Mol) Kaliumnitrat zu und lässt man innerhalb von 75 Stunden bei einer Temperatur von 20°C stehen. Dann wird die Reaktionsmasse auf Eis ausgegossen, der Niederschlag wird abgefiltert, mit Wasser und mit 5%iger Sodalösung und mit Wasser gewaschen und getrocknet. Man erhält 2,I2 g (75,1%) 2-Brom-4,6-dinitrochlorbenzol).

Beispiel I5

Einer Lösung aus 0,78 g (0,005 Mol) p-Nitrochlorbenzol in 40 ml Schwefelsäure (d = I,83) setzt man bei einer Temperatur von 40 bis 50°C 0,8 g (0,005 Mol) Brom und 2,02 g (2,02 Mol) Kaliumnitrat zu, und lässt innerhalb von 20 Minuten stehen und erwärmt man auf I20°C. Bei dieser Temperatur lässt man das Gemisch innerhalb von 2,5 Stunden stehen, dann kühlt man ab und giesst auf Eis aus. Der Niederschlag wird abgefiltert, mit Wasser, mit 5%iger Sodalösung und mit Wasser gewaschen und getrocknet. Man erhält I,38 g (99,0%) 2-Brom-4,6-dinitrochlorbenzol.

Beispiel 16

Einer Lösung aus 47,25 g (0,3 Mol) p-Nitrochlorbenzol in I00 ml Schwefelsäure (d = I,83) bei einer Temperatur von 40 bis 50°C setzt man 26,4 g (0,I5 Mol) Brom und 64,2 g (0,75 Mol) Natriumnitrat zu, lässt innerhalb von 20 Minuten stehen, erwärmt man auf 70°C und lässt bei dieser Temperatur innerhalb von 30 Stunden stehen. Die Isolierung des Produktes erfolgt analog Beispiel I5. Man erhält 80,73 g (95,6%) 2-Brom-4,6-dinitrochlorbenzol.

Beispiel 17

Einer Lösung aus 2,05 g (0,0I3 Mol) p-Nitrochlorbenzol in 40 ml Schwefelsäure (d = I,83) setzt man bei einer Temperatur von 40 bis 50°C I,I2 g (0,007 Mol) Brom und 6,59 g (0,065 Mol) Kaliumnitrat zu, lässt innerhalb von 20 Minuten stehen und erwärmt man auf 70°C, dann lässt man bei dieser Temperatur weitere 6 Stunden stehen. Man isoliert das Produkt unter den in Beispiel I5 genannten analogen Bedingungen. Man erhält 3,62 g (98,9%) 2-Brom-4,6-dinitrochlorbenzol.

Beispiel 18

Einer Lösung aus 0,78 g (0,005 Mol) p-Nitrochlorbenzol in 20 ml Schwefelsäure (d = 1,83) setzt man bei einer Temperatur von 40 bis 50°C 0,8 g (0,005 Mol) Brom und 1,00 g (0,01 Mol) Kaliumnitrat zu, lässt innerhalb von 20 Minuten stehen und erwärmt auf 80°C, dann lässt man bei dieser Temperatur weitere 25 Stunden stehen. Man scheidet ein Produkt unter den in Beispiel 15 genannten analogen Bedingungen aus. Man erhält 1,03 g eines Gemisches aus 2,4-Dinitrochlorbenzol mit 2-Brom-4,6-dinitrochlorbenzol.

Beispiel 19

Einer Lösung aus 1,56 g (0,01 Mol) p-Nitrochlorbenzol in 40 ml Schwefelsäure (d = 1,83) setzt man bei einer Temperatur von 40 bis 50°C 3,58 g (0,03 Mol) Kaliumbromid und 1,00 ml (0,025 Mol) Salpetersäure (d = 1,505) zu und lässt bei einer Temperatur von 20°C innerhalb von 70 Stunden stehen. Man isoliert ein Produkt unter den in Beispiel 15 genannten analogen Bedingungen. Man erhält 2,14 g (70,0%) 2-Brom-4,6-dinitrochlorbenzol.

Beispiel 20

Einer Lösung aus 0,78 g (0,005 Mol) p-Nitrochlorbenzol in 40 ml Schwefelsäure (d = 1,83) setzt man bei einer Temperatur von 40 bis 50°C 1,20 g (0,01 Mol) Kaliumbromid und 0,65 ml (0,015 Mol) Salpetersäure (d = 1,505) zu, lässt innerhalb von 20 Minuten stehen und erwärmt auf 120°C, dann lässt man bei dieser Temperatur innerhalb von 2 Stunden stehen, aus der Reaktionsmasse isoliert man das Produkt unter den in Beispiel 15 genannten analogen Bedingungen. Man erhält 1,39 g (99,7%) 2-Brom-4,6-dinitrochlorbenzol.

Beispiel 21

Einer Lösung aus 47,25 g (0,3 Mol) p-Nitrochlorbenzol in 100 ml Schwefelsäure (d = 1,83) setzt man bei einer Temperatur von 40 bis 50°C 34,0 g (0,3 Mol) Natriumbromid und 26,02 ml (0,6 Mol) Salpetersäure (d = 1,505) zu, lässt innerhalb von 20 Minuten stehen und erwärmt auf 70°C, dann lässt man bei dieser Temperatur innerhalb von 25 Stunden stehen. Man isoliert ein Produkt unter den in Beispiel 15 genannten analogen Bedingungen. Man erhält 83,42 g (98,8%)

2-Brom-4,6-dinitrochlorbenzol.

Beispiel 22

Einer Lösung aus 2,05 g (0,0I2 Mol) p-Nitrochlorbenzol in 40 ml Schwefelsäure (d = I,83) setzt man bei einer Temperatur von 40 bis 50°C I,55 g (0,0I3 Mol) Kaliumbromid und 2,27 ml (0,052 Mol) Salpetersäure (d = I,505) zu, lässt innerhalb von 20 Minuten stehen und erwärmt auf 70°C, dann lässt man bei dieser Temperatur weitere 6 Stunden stehen. Aus der Reaktionsmasse wird das Produkt unter den in Beispiel I5 genannten analogen Bedingungen isoliert. Man erhält 3,64 g (99,5%) 2-Brom-4,6-dinitrochlorbenzol.

Beispiel 23

Einer Lösung aus 0,78 g (0,005 Mol) p-Nitrochlorbenzol in 20 ml Schwefelsäure (d = I,83) setzt man bei einer Temperatur von 40 bis 50°C 0,59 g (0,005 Mol) Kaliumbromid und 0,3 ml (0,0075 Mol) Salpetersäure (d = I,505) zu, lässt innerhalb von 20 Minuten stehen und erwärmt auf 80°C, dann lässt man bei dieser Temperatur weitere 35 Stunden stehen. Man isoliert das Produkt unter den in Beispiel I5 genannten analogen Bedingungen. Man erhält I,07 g eines Gemisches aus 2-4-Dinitrochlorbenzol und 2-Brom-4,6-dinitrochlorbenzol.

Beispiel 24

Einer Lösung aus I,56 g (0,0I Mol) p-Nitrochlorbenzol in 40 ml Schwefelsäure (d = I,83) setzt man bei einer Temperatur von 40 bis 50°C 3,58 g (0,03 Mol) Kaliumbromid und 3,24 g (0,033 Mol) Kaliumnitrat zu und lässt bei einer Temperatur von 20°C innerhalb von 75 Stunden stehen. Dann isoliert man das Produkt unter den in Beispiel I5 genannten analogen Bedingungen. Man erhält 2,03 g (73,0%) 2-Brom-4,6-dinitrochlorbenzol.

Beispiel 25

Einer Lösung aus 0,78 g (0,005 Mol) p-Nitrochlorbenzol in 40 ml Schwefelsäure (d = I,83) setzt man bei einer Temperatur von 40 bis 50°C 1,20 g (0,0I Mol) Kaliumbromid und 2,02 g (0,02 Mol) Kaliumnitrat zu, lässt innerhalb von 20 Minuten stehen und erwärmt man auf I20°C. Dann lässt man bei dieser Temperatur weitere 2,5 Stunden stehen und isoliert

man ein Produkt unter den in Beispiel I5 genannten analogen Bedingungen aus. Man erhält I,35 g (96,8%) 2-Brom-4,6-dinitrochlorbenzol.

Beispiel 26

Einer Lösung aus 47,25 g (0,3 Mol) p-Nitrochlorbenzol in I00 ml Schwefelsäure (d = I,83) setzt man bei einer Temperatur von 40 bis 50°C 34,0 g (0,3 Mol) Natriumbromid und 64,2 g (0,75 Mol) Natriumnitrat zu, lässt innerhalb von 20 Minuten stehen und erwärmt auf 70°C, dann lässt man bei dieser Temperatur weitere 30 Stunden stehen. Danach isoliert man das Produkt unter den in Beispiel I5 genannten analogen Bedingungen. Man erhält 75,8 g (89,7%) 2-Brom-4,6-dinitrochlorbenzol.

Beispiel 27

Einer Lösung aus 2,05 g (0,0I3 Mol) p-Nitrochlorbenzol in 40 ml Schwefelsäure (d = I,83) setzt man bei einer Temperatur von 40 bis 50°C I,55 g (0,0I3 Mol) Kaliumbromid und 6,59 g (0,065 Mol) Kaliumnitrat zu, lässt innerhalb von 20 Minuten stehen und erwärmt auf 70°C, dann lässt man bei dieser Temperatur weitere 6 Stunden stehen. Aus der Reaktionsmasse isoliert man das Produkt unter den in Beispiel I5 genannten analogen Bedingungen. Man erhält 3,58 g (97,8%) 2-Brom-4,6-dinitrochlorbenzol.

Beispiel 28

Einer Lösung aus 0,78 g (0,005 Mol) p-Nitrochlorbenzol in 20 ml Schwefelsäure (d = I,83) setzt man bei einer Temperatur von 40 bis 50°C 0,59 g (0,005 Mol) Kaliumbromid und I,00 g (0,0I Mol) Kaliumnitrat zu, lässt innerhalb von 20 Minuten stehen und erwärmt auf 80°C, dann lässt man bei dieser Temperatur weitere 35 Stunden stehen. Aus der Reaktionsmasse isoliert man das Produkt unter den in Beispiel I5 genannten analogen Bedingungen. Man erhält 0,96 g eines Gemisches aus 2,4-Dinitrochlorbenzol mit 2-Brom-4,6-dinitrochlorbenzol.

Gewerbliche Verwertbarkeit

Die Erfindung wird bei der Produktion solcher Produkte wie Farbstoffe, beispielsweise, disperse und Metallkomplexfarbstoffe; Pigmente, Herbizide, Fungizide, Antioxydations-

mittel, das heisst solcher Produkte, bei deren Synthese man als Ausgangsprodukt das 2-Brom-4,6-dinitrochlorbenzol verwendet, eine umfassende Anwendung finden.

PATENTANSPRÜCHE:

I. Verfahren zur Herstellung von 2-Brom-4,6-dinitrochlorbenzol, ausgehend von einer aromatischen Halogenderivat-Verbindung, dadurch g e k e n n z e i c h n e t, dass man als aromatische Halogenderivat-Verbindung Chlorbenzol, das vorher mit einem Gemisch der Schwefel- und Salpetersäure behandelt wird, oder individuelle o-, p-Nitrochlorbenzole, oder ein eutektisches Gemisch der o-, p-, m-Nitrochlorbenzole verwendet, die man einer Behandlung mit Brom oder mit Alkalimetallbromid, mit Salpetersäure oder mit Alkalimetallnitrat und mit Schwefelsäure oder mit Oleum bei folgenden Molverhältnissen der Reagenzien aussetzt: die genannte aromatische Halogenderivat-Verbindung: Brom oder Alkalimetallbromid: Salpetersäure oder Alkalimetallnitrat: Schwefelsäure oder Oleum gleich I,0:0,5-I,5 oder I,0-3,0:2,0-4,0 oder 2,5-5,0: 6,0-70,0, bei einer Temperatur von 20 bis I20°C mit anschliessender Isolierung des Endproduktes.

2. Verfahren nach Anspruch I, dadurch g e k e n n z e i c h n e t, dass man bei der Verwendung von Chlorbenzol als Ausgangsverbindung dieses der Behandlung mit einem Gemisch aus Salpeter- und Schwefelsäure bei ihrem Molverhältnis I,0:I,I:0,9 mit anschliessender Behandlung des angefallenen Reaktionsgemisches mit einem Gemisch aus Oleum, Brom und Salpetersäure bei ihrem Molverhältnis I,0:6,9:0,5:2,0 aussetzt.

3. Verfahren nach Anspruch I, dadurch g e k e n n z e i c h n e t, dass man bei Verwendung von p-Nitrochlorbenzol dieses mit einem Gemisch aus Schwefelsäure, Brom und Salpetersäure bei ihrem Molverhältnis I,0:70,0:I,5:2,5 behandelt.

4. Verfahren nach Anspruch I, dadurch g e k e n n z e i c h n e t, dass man bei Verwendung von o-Nitrochlorbenzol dieses mit einem Gemisch aus Schwefelsäure, Brom und Salpetersäure bei ihrem Molverhältnis I,0:58,0:0,5:2,0 behandelt.

5. Verfahren nach Anspruch I, dadurch g e k e n n z e i c h n e t, dass man bei Verwendung eines eutektischen Gemisches von o-, p-, m-Nitrochlorbenzolen dieses mit einem Gemisch aus Schwefelsäure, Brom und Salpetersäure bei ihrem

Molverhaltnis I,0:59,0:0,5:2,0 behandelt.

# INTERNATIONAL SEARCH REPORT

International Application No PCT/SU 89/00331

**I. CLASSIFICATION OF SUBJECT MATTER** (if several classification symbols apply, indicate all) [6]

According to International Patent Classification (IPC) or to both National Classification and IPC

Int.Cl.$^5$ C07C 205/I2,20I/08,20I/06,20I/14,C07B 39/00,43/02

**II. FIELDS SEARCHED**

Minimum Documentation Searched [7]

| Classification System | Classification Symbols |
|---|---|
| Int.Cl.$^4$ | C07C 79/I2, C07B, 39/00 42/02 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No [13] |
|---|---|---|
| A | DE,B2,2001570 (BAYER A G ),20 July 1978 (20.07.78),see claims | I-5 |
| A | DE,BI,2555736 (BASE A G),16 June 1977 (16.06.77),see claims | I-5 |
| A | DE,B2;2657234 (BASE A G),22 November 1979 (22.11.79), see claims | I-5 |
| A | US,A,4418228 (GULE RESEARCH & DEVELOPMENT COMPANY),29 November 1983 (29.11.83), see claims | I-5 |
| A | DD,A,204246 (VEB CHEMIEKOMBINAT BITTERFELD) 23 November1983 (23.11.83), see claims | I-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 22 February 1990 (22.02.90) | 2 April 1990 (02.04.90) |
| **International Searching Authority** | **Signature of Authorized Officer** |
| ISA/SU | |